# EUROPEAN PATENT SPECIFICATION

(11) **EP 3 888 091 B1**
(45) Date of publication and mention of the grant of the patent: **28.02.2024**
(21) Application number: 19817177.9
(22) Date of filing: 29.11.2019
(51) Int. Cl.: G16B 15/30, G16B 40/20

(54) **MACHINE LEARNING FOR PROTEIN BINDING SITES**
MASCHINELLES LERNEN FÜR PROTEINBINDUNGSSTELLEN
APPRENTISSAGE MACHINE POUR L'IDENTIFICATION DE SITES DE LIAISON PROTÉINIQUE

(30) Priority: 29.11.2018 GB 201819498
(43) Date of publication of application: 06.10.2021
(73) Proprietor: BenevolentAI Technology Limited, London Greater London W1T 5HD (GB)
(72) Inventor: MEYERS, Joshua, London Greater London W4 3EX (GB); SEGLER, Marwin, Hampshire PO5 2PR (GB); SIMONOVSKY, Martin, 69115 Heidelberg (DE)
(74) Representative: Gill Jennings & Every LLP
(86) International application number: PCT/EP2019/083188
(87) International publication number: WO 2020/109608

(56) References cited:
- MICHAL BRYLINSKI: "eMatchSite: Sequence Order-Independent Structure Alignments of Ligand Binding Pockets in Protein Models", PLOS COMPUTATIONAL BIOLOGY, vol. 10, no. 9, 18 September 2014 (2014-09-18), page e1003829, XP055672342, DOI: 10.1371/journal.pcbi.1003829
- MISAGH NADERI ET AL: "Binding site matching in rational drug design: algorithms and applications", BRIEFINGS IN BIOINFORMATICS., vol. 20, no. 6, 31 August 2018 (2018-08-31), pages 2167-2184, XP055672312, GB ISSN: 1467-5463, DOI: 10.1093/bib/bby078

## Description

The present application relates to a system and method for using machine learning to identify characteristics of protein binding sites. In particular, the application relates to using machine learning techniques to determine whether two binding sites are related to each other, for example by virtue of binding the same ligand.

### Background

A range of machine learning techniques have been developed in the field of drug discovery to assess proteins and ligand binding characteristics. For example, machine learning has been used to assess the structural characteristics of proteins and to identify regions of proteins that are likely to be binding sites for a ligand or for another protein.

Known techniques help address some of the questions that arise in drug discovery, such as will this protein will bind a ligand, what other proteins will bind the same ligand, and what is the function of this protein?

Michal Brylinski: "Sequence Order-Independent Structure Alignments of Ligand Binding Pockets in Protein Models" provides eMatchSite, a new method for constructing sequence order-independent alignments of ligand binding sites in protein models. Large-scale benchmarking calculations using adenine-binding pockets in crystal structures demonstrate that eMatchSite generates accurate alignments for almost three times more protein pairs than SOIPPA. More importantly, eMatchSite offers a high tolerance to structural distortions in ligand binding regions in protein models.

Misagh Naderi ET Al: "Binding site matching in rational drug design: algorithm and applications" is a survey of 12 tools widely used to match pockets. These methods are divided into five categories based on the algorithm implemented to construct binding-site alignments. In addition to the comprehensive analysis of their algorithms, test sets and the performance of each method are described. We also discuss general pharmacological applications of computational pocket matching in drug repurposing, polypharmacology and side effects. Reflecting on the importance of these techniques in drug discovery, in the end, we elaborate on the development of more accurate meta-predictors, the incorporation of protein flexibility and the integration of powerful artificial intelligence technologies such as deep learning.

However, the extent to which known techniques can answer these questions is limited.

The embodiments described below are not limited to implementations which solve any or all of the disadvantages of the known approaches described above.

### Summary of the invention

The invention is set out in the appended set of claims.

### Summary of the disclosure

This Summary is provided to introduce a selection of concepts in a simplified form that are further described below in the Detailed Description. This Summary is not intended to identify key features or essential features of the claimed subject matter, nor is it intended to be used to determine the scope of the claimed subject matter.

In a first aspect, the present disclosure provides a computer-implemented method of training a machine learning model to learn ligand binding similarities between protein binding sites, the method comprising: inputting to the machine learning model: a representation of a first binding site; a representation of a second binding site, wherein the representations of the first and second binding sites comprise structural information; and a label comprising an indication of ligand binding similarity between the first binding site and the second binding site; outputting from the machine model a similarity indicator based on the representations of the first and second binding sites; performing a comparison between the similarity indicator and the label; and updating the machine learning model based on the comparison.

The structural information relates to three-dimensional structure of the binding sites.

The structural information comprises volumetric information.

The representations of the first and second binding sites each comprise an encoded three-dimensional grid of voxels, each voxel being associated with an occupancy value indicating whether an atom is present.

Optionally, wherein each voxel is associated with a further value indicating a property selected from the set of hydrophobicity, aromaticity, acceptance or donation of a hydrogen bond, positive or negative ionizability, and being metallic.

Optionally, wherein the machine learning model comprises a neural network.

Optionally, the neural network comprises one or more convolutional layers.

Optionally, the neural network comprises one or more max-pooling layers.

Optionally, the neural network comprises a steerable three-dimensional convolutional neural network.

Optionally, the neural network comprises a deep learning neural network.

Optionally, performing the comparison comprises minimising a loss function.

Optionally, updating the machine learning model comprises performing back propagation using the minimised loss function.

Optionally, the loss function comprises a contrastive loss representing a loss between the similarity indicator and the label.

Optionally, the loss function comprises a triplet loss based on a pair of binding sites, a reference binding site and the label.

Optionally, the method comprises jittering the binding sites in input space.

Optionally, the label comprises a binary value indicating whether the first and second binding sites bind structurally similar ligands.

In a second aspect, the present disclosure provides a neural network model obtained from a computer implemented method according to the first aspect.

In a third aspect, the present disclosure provides a computer-implemented method of using a neural network model, wherein the neural network model is obtained from a computer implemented method according to the first aspect, the method of using the neural network model comprising: inputting to the neural network model respective representations of third and fourth binding sites; and using the neural network model to output a ligand binding similarity indicator.

Optionally, the ligand binding similarity indicator comprises an indication of whether the first and second binding sites are likely to bind structurally similar ligands.

In a fourth aspect, the present disclosure provides an apparatus comprising a processor, a memory unit and a communication interface, wherein the processor is connected to the memory unit and the communication interface, wherein the processor and memory are configured to implement the computer-implemented method according to any one of the first, second and third aspects.

In a fifth aspect, the present disclosure provides a computer-readable medium comprising data or instruction code representative of a machine learning model generated according to the method of any one of the first, second and third aspects, which when executed on a processor causes the processor to implement the machine learning model.

In a sixth aspect, the present disclosure provides a computer-readable medium comprising data or instruction code which, when executed on a processor, causes the processor to implement the computer-implemented method of any one of the first, second and third aspects.

The methods described herein may be performed by software in machine readable form on a tangible storage medium e.g. in the form of a computer program comprising computer program code means adapted to perform all the steps of any of the methods described herein when the program is run on a computer and where the computer program may be embodied on a computer readable medium. Examples of tangible (or non-transitory) storage media include disks, thumb drives, memory cards etc. and do not include propagated signals. The software can be suitable for execution on a parallel processor or a serial processor such that the method steps may be carried out in any suitable order, or simultaneously.

This application acknowledges that firmware and software can be valuable, separately tradable commodities. It is intended to encompass software, which runs on or controls "dumb" or standard hardware, to carry out the desired functions. It is also intended to encompass software which "describes" or defines the configuration of hardware, such as HDL (hardware description language) software, as is used for designing silicon chips, or for configuring universal programmable chips, to carry out desired functions.

The preferred features may be combined as appropriate, as would be apparent to a skilled person, and may be combined with any of the aspects of the invention.

### Brief Description of the Drawings

Embodiments of the invention will be described, by way of example, with reference to the following drawings, in which:
Figure 1A is a perspective view of part of a first protein-ligand complex comprising the protein kinase PLK1 and the ligand BI-2536;
Figure 1B is a perspective view of part of a second protein-ligand complex comprising the protein bromodomain BRD4 and the ligand BI-2536;
Figure 1C is a perspective view of an overlay of the first and second protein-ligand complexes;
Figure 2 is a block diagram showing a system for training a neural network to learn ligand binding similarities between protein binding sites;
Figure 3 is a flow chart showing a method of training a neural network to learn ligand binding similarities between protein binding sites;
Figure 4 is a block diagram showing an example network architecture of the neural network of Figure 3;
Figure 5 is a block diagram showing a neural network model for predicting ligand binding similarities between protein binding sites, the neural network model having been trained according to the method of Figure 3; and
Figure 6 is a block diagram showing a computer suitable for implementing the above training or use of a neural network.

Common reference numerals are used throughout the figures to indicate similar features.

### Detailed Description

Embodiments of the present invention are described below by way of example only. These examples represent the best ways of putting the invention into practice that are currently known to the Applicant although they are not the only ways in which this could be achieved. The description sets forth the functions of the example and the sequence of steps for constructing and operating the example. However, the same or equivalent functions and sequences may be accomplished by different examples.

In known techniques, geometric algorithms are used to assess similarity between binding sites based on their three-dimensional structure. Such techniques use geometrically based methods that require drug discovery scientists to make assumptions about which structural features are important for assessing similarity, and as such are based on the intuition of the drug discovery scientist. These intuition-based expectations introduce bias into subsequent inferences and therefore place restrictions on their ability to accurately identify binding sites with similar ligand binding characteristics.

Part of the danger of making assumptions about which structural features are relevant for assessing ligand binding similarity is that some pairs of binding sites may bind the same ligand despite looking quite different to the human observer.

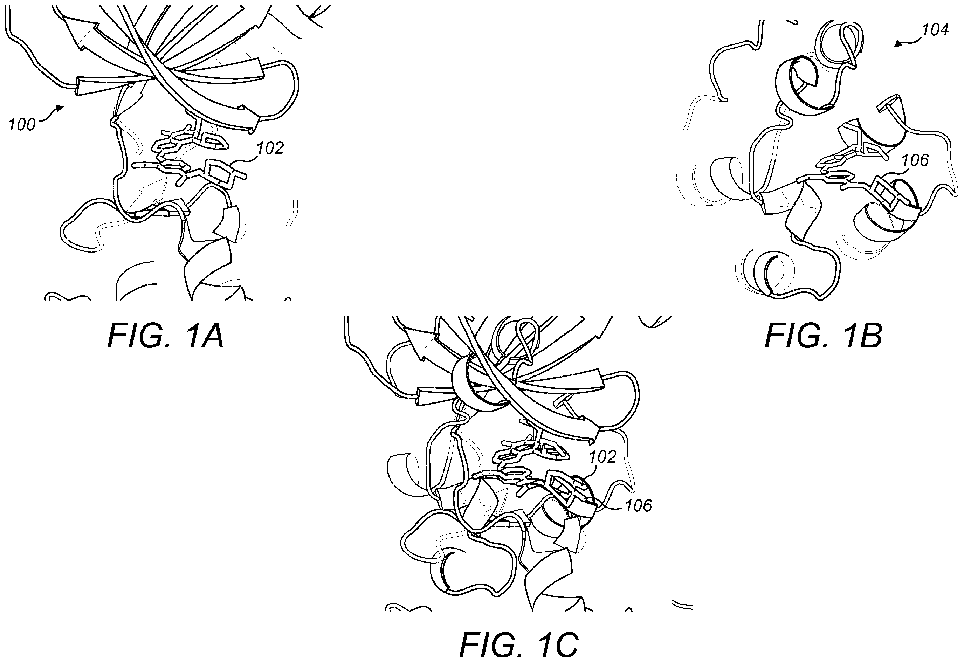

This is demonstrated in Figures 1A and 1B which show two different protein-ligand complexes involving a common ligand. In Figure 1A, a binding site 100 of the protein kinase PLK1 is shown binding a ligand 102, BI-2536, while Figure 1B shows a binding site 104 of the protein bromodomain BRD4 binding a ligand 106, B12536. While the ligands 102, 106 are of the same type (B12536), it can be appreciated from Figures 1A and 1B that the structures of the binding sites 100 and 104 have quite different features, and even their overall shapes do not look strikingly similar. Based the structural differences it may be surprising that the binding sites 100, 104 can bind the same ligand, yet they do. The binding site structures can be more directly compared with reference to Figure 1C which shows the binding site 100 and ligand 102 of Figure 1A overlaid on the binding site 104 and ligand 106 of Figure 1B.

A technique is required that does not rely on intuition-based assumptions about which structural features are important for assessing ligand binding similarities between binding sites. With this in mind, a system for training a machine learning model such as a neural network to learn relatedness such as ligand binding similarities between protein binding sites will be disclosed. In this document, the term 'protein binding site' is used interchangeably with 'binding site' and both refer to a region on a protein that is capable of binding an entity of interest such as a ligand (including a synthetic or endogenous ligand), a biomolecule, or a material. Protein binding sites are also commonly referred to as 'pockets' in the literature.

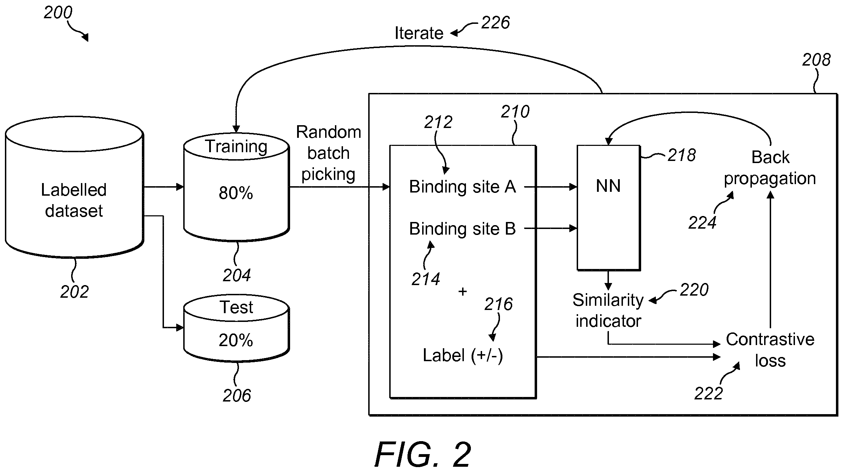

Figure 2 shows an example system 200 for training a neural network to learn ligand binding similarities between protein binding sites. Rather than relying on drug discovery scientists to identify structural features of binding sites that are relevant for assessing similarity, the system 200 uses a labelled dataset 202 that includes functional information comparing ligand binding abilities along with binding site structures.

In the system 200, the labelled dataset 202 is split in two with 80% being used as training data 204 and 20% as test data 206. It will be appreciated that although these proportions are commonly used in machine learning, other proportions would also be suitable. The training data 204 is provided to a training module 208 either as individual items of training data or in batches of items, in which case a random batch picking tool may be used to select batches from the training set 204

An item 210 of training data which may belong to a batch is shown inside the training module 208 and comprises three pieces of information: a representation 212 of a first binding site A, a representation 214 of a second binding site B, and a label 216 indicating whether the first and second binding sites bind the same ligand. In other examples, the label may comprise a score indicating a likelihood that the binding sites bind the same ligand, but in the example system 200 of Figure 2 the label is binary and indicates a yes or no answer. In general, the label comprises data relating to whether or not two binding sites are related to each other. They may be related for example by virtue of binding the same ligand, binding a same protein or nucleotide, having a common functionality, or belonging to proteins in a same class of proteins such as a same evolutionary class. While the label may provide functional information indicating similarities in ligand binding and other functionalities, the representations 212 and 214 comprise structural information relating to the three-dimensional structure of the binding sites. For example, the structural information may take the form of volumetric representations of the binding sites, as described below.

The training module 208 is configured to provide the representations 212, 214 of binding sites A and B to a neural network 218 which projects them into a latent space and outputs a similarity indicator 220 that provides an indication of similarity or distance between the binding sites. The similarity indicator 220 may for example be based on a distance between the binding sites in a Euclidean latent space.

At this stage there are two descriptions of how similar the binding sites are. Firstly, the similarity indicator 220 indicates how far apart the binding site representations are when they have been projected into latent space. Since this is based on the representations 212, 214 which comprise structural information, these relate to structural similarity. Secondly, the label 216 indicates whether or not the binding sites bind the same ligand, and as such relates to functional similarity. To combine these, the training module is configured to minimise a contrastive loss function 222 for the two binding sites based on the similarity indicator 220 and the label 216, and to update the neural network 218 by back propagation 224 using the minimised contrastive loss 222.

The training process is iterated 226 until convergence when the contrastive loss 222 stops decreasing.

Although the system 200 of Figure 2 uses a neural network, it will be appreciated that other example systems could use other machine learning models such as Random Forests, Support Vector Machines, or approaches for metric learning such as Linear Discriminant Analyses. Furthermore, other example systems may use other methods to compare the similarity indicator and the label rather than by using a contrastive loss function. For example, other functions such as hinge, softmin, cross-entropy loss, or a triplet loss function (described below) could be used.

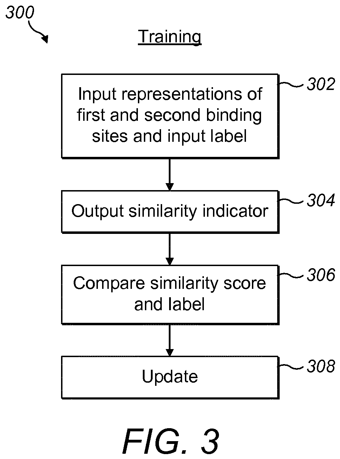

As such, and with reference to Figure 3, a computer-implemented method 300 of training a machine learning model to learn ligand binding similarities between protein binding sites is disclosed. The method comprises: inputting 302 to the machine learning model: a representation of a first binding site; a representation of a second binding site, wherein the representations of the first and second binding sites comprise structural information; and a label comprising an indication of ligand binding similarity between the first binding site and the second binding site; outputting 304 from the machine model a similarity indicator based on the representations of the first and second binding sites; performing a comparison 306 between the similarity indicator and the label; and updating 308 the machine learning model based on the comparison.

The representations of binding sites may comprise structural information expressed in terms of three-dimensional volumes. For example, a representation of a binding site may be generated by converting a three dimensional image of the binding site into a grid of voxels. In this case, each voxel may be associated with a set of values providing information about the part of the binding site at that location. For example, occupancy provides an indication of whether or not an atom is present at a location and may take values of 0 or 1. In this case, occupancy information may be based on a smooth function of Van der Waals radii of atoms. Other classes of information or 'feature channels' may be provided, such as hydrophobicity, aromaticity, acceptance or donation of a hydrogen bond, positive or negative ionizability, and being metallic. Taking these seven pharmacophoric properties together with occupancy gives a total of 8 feature channels in this example.

A suitable arrangement may for example comprise each voxel being a cubic angstrom and the grid being a cube having 24 voxels along each side and being centred around a geometric centre of the binding site.

In order to provide the grid of voxels, each having 8 associated values, into a format suitable to be inputted into a machine learning model such as a neural network, the grid of voxels may be flattened into an ordered array of values.

The comparison between the similarity indicator and the label may comprise using a loss function that operates on pairs or triplets of projected binding sites. Contrastive loss functions operate on pairs of projected binding sites and use a similarity indicator that represents a distance between two binding sites in latent space. Triplet loss functions operate on triplets of projected binding sites and use a similarity indicator having two portions: a first portion representing a distance between a first binding site and a reference binding site in latent space and a second portion representing a distance between a second binding site and the reference binding site in latent space. As such, contrastive loss functions work on the basis of comparing binding sites in latent space directly amounting to an absolute distance evaluation, whereas triplet loss functions compare binding sites in latent space indirectly amounting to a relative distance evaluation.

For the purpose of comparing protein binding sites, it is preferable to use a contrastive loss function because triplet loss functions require intersecting pairs of binding sites in the training data to supply reference binding sites. For example, if the training dataset comprises labelled binding site pairs A-B, B-C and C-D, then information can be inferred regarding the pairs A-C, A-D and B-D because the labelled pairs comprise binding sites in common (i.e. there are intersecting labelled pairs). However, if the training dataset comprises labelled binding site pairs A-B and C-D, there are no intersecting pairs in the training dataset, so information cannot be inferred regarding the pairs A-D or B-C. In practice, having too few intersecting pairs in the training dataset can restrict the availability of reference binding sites and therefore create problems when using a triplet loss function.

When using a loss function to compare a similarity indicator and a label, the similarity indicator can be brought into conformity with the label by minimising the loss function. For example, in the example system 200 of Figure 2, the label takes binary values of 0 and 1 indicating whether or not two binding sites bind the same ligand. As a result, if a label takes a value of 0 indicating that the binding sites do not bind the same ligand, then the loss function may require the projected binding sites to be separated by a minimum distance in latent space. On the other hand, if a label takes a value of 1 indicating that the binding sites bind the same ligand, then the loss function may require the projected binding sites to be colocated in latent space.

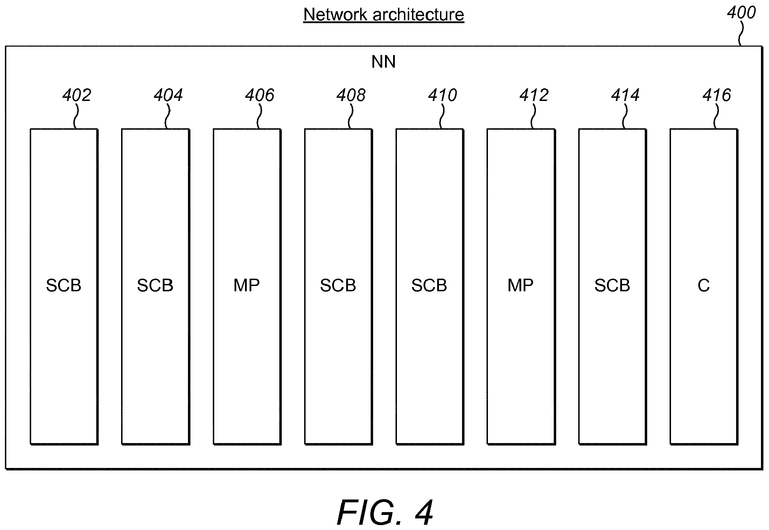

In implementations where the machine learning model comprises a neural network, the neural network may comprise a three-dimensional convolutional neural network (3D CNN) such as the network 400 shown in Figure 4. Network 400 has eight layers shown in left to right order in Figure 4, comprising a total of five steerable convolutional blocks, two max pooling layers, and one standard convolution as follows.

| | |
|---|---|
| Layer 402 | Steerable convolution block |
| Layer 404 | Steerable convolution block |
| Layer 406 | Max pooling layer |
| Layer 408 | Steerable convolution block |
| Layer 410 | Steerable convolution block |
| Layer 412 | Max pooling layer |
| Layer 414 | Steerable convolution block |
| Layer 416 | Standard convolution |

Other suitable 3D CNNs may comprise different numbers of layers in a different order to the example of Figure 4. However, it is preferable that they comprise one or more steerable convolutional blocks to ensure that projected binding sites are rotationally invariant as well as translationally invariant. The rotational invariance is advantageous because the task of representing protein binding sites is rotationally invariant. The approach of using a steerable CNN is preferable over traditional techniques that attempt to provide rotational invariance using strong data augmentation and overparameterisation of models. The network 400 of Figure 4 provides a translationally and rotationally invariant descriptions of binding sites in latent space while using comparatively few model parameters on the order of 10⁵.

Data augmentation techniques such as any one or more of the following three may be used during training of the machine learning model. Firstly, binding site locations in input space may be translated or 'jittered', for example by up to 2 angstroms, in order to account for uncertainty in the precise locations of the centres of binding sites. Uncertainty may arise from variation in the ways in which the centre or 'centroid' of a binding site may be defined. For example, a binding site centroid may be defined by the location of a bound ligand that has crystallised in a particular position in a protein-ligand complex. However, the same ligand could have crystallised in a slightly different position due to molecular dynamics, and furthermore the same binding site may also bind other ligands giving a slightly different centroid location. Binding site centroids may also be defined using binding site detection algorithms (commonly referred to as 'pocket detection algorithms'), which may generate slightly different definitions of the location of the binding site centroid. The jittering of the centroids in input space is intended to simulate the influence of these factors on the geometric centre of a binding site and ensure that the machine learning model does not overfit onto a particular centroid for a binding site.

Secondly, proteins are randomly rotated before being represented as a grid of discrete voxels. This varies the voxel boundaries with respect to the protein, so that atoms A and B, which may be defined by smooth Van der Waals functions, may be located in the same voxel before the rotation but in neighbouring voxels after the rotation. The rotation ensures that the machine learning model does not overfit onto a particular discretised representation of the binding site, and thereby gains invariance to discretisation artifacts.

Thirdly, random points in protein representations having a low probability such as 0.1 of being a binding site centroid are sampled and used to train the machine learning model. This increases the variability of the training data for negative pairs (i.e. inputs that do not represent related or similar binding sites) and enables the model to recognise regions of proteins that are not binding sites. As a result, the model is able to predict accurate, low similarity when non-binding site inputs from elsewhere in the protein are provided.

When training the machine learning model, for example the neural network 218 of system 200 shown in Figure 2, batches of 64 pairs of binding sites may be used. In this case, it would be suitable for example to use the 16 highest loss pairs that bind the same ligand (i.e. the 16 highest loss positive pairs) and the 16 highest loss pairs that do not bind the same ligand (i.e. the 16 highest loss negative) and update the model parameters, for example by performing backpropagation in the case of the neural network 218, using only those 32 highest loss pairs.

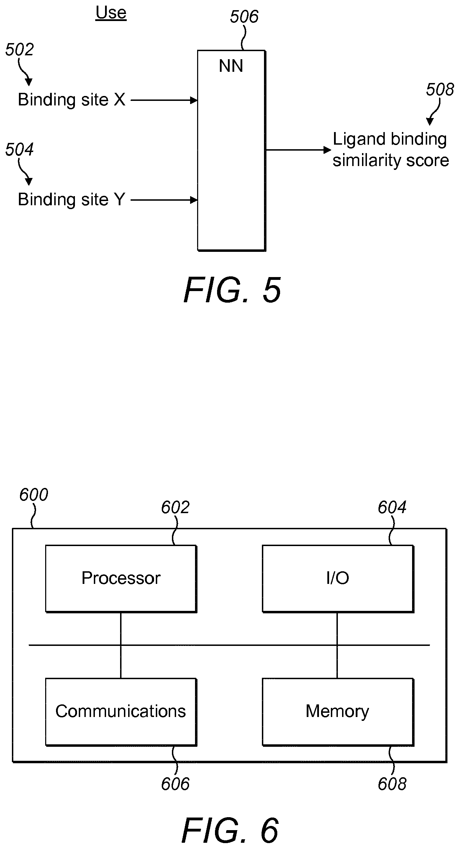

Use of a neural network model 506 trained in accordance with the present disclosure is shown in Figure 5. A representation 502 of a binding site X is provided together with a representation 504 of a binding site Y to the model 506. The model 506 is configured to predict a ligand binding similarity indicator 508 indicating whether or not binding sites X and Y bind the same ligand. The model 506 trained according to the present disclosure provides improved rates of accurate predictions of whether or not binding sites bind the same ligand. This has positive implications for fields such as drug discovery where such information helps to answer questions such as which ligands bind a particular protein, and what is the function of a particular protein.

Figure 6 shows hardware 600 suitable for implementing the methods of the present disclosure. The hardware 600 comprises a processor 602, an input-output module 604, a communications module 606, and memory 608. For example, the memory 608 may store computer code that when executed using the processor 602 causes the computer hardware to perform a method according to the present disclosure.

In the embodiment described above the server may comprise a single server or network of servers. In some examples the functionality of the server may be provided by a network of servers distributed across a geographical area, such as a worldwide distributed network of servers, and a user may be connected to an appropriate one of the network of servers based upon a user location.

The above description discusses embodiments of the invention with reference to a single user for clarity. It will be understood that in practice the system may be shared by a plurality of users, and possibly by a very large number of users simultaneously.

The embodiments described above are fully automatic. In some examples a user or operator of the system may manually instruct some steps of the method to be carried out.

In the described embodiments of the invention the system may be implemented as any form of a computing and/or electronic device. Such a device may comprise one or more processors which may be microprocessors, controllers or any other suitable type of processors for processing computer executable instructions to control the operation of the device in order to gather and record routing information. In some examples, for example where a system on a chip architecture is used, the processors may include one or more fixed function blocks (also referred to as accelerators) which implement a part of the method in hardware (rather than software or firmware). Platform software comprising an operating system or any other suitable platform software may be provided at the computing-based device to enable application software to be executed on the device.

Various functions described herein can be implemented in hardware, software, or any combination thereof. If implemented in software, the functions can be stored on or transmitted over as one or more instructions or code on a computer-readable medium. Computer-readable media may include, for example, computer-readable storage media. Computer-readable storage media may include volatile or non-volatile, removable or non-removable media implemented in any method or technology for storage of information such as computer readable instructions, data structures, program modules or other data. A computer-readable storage media can be any available storage media that may be accessed by a computer. By way of example, and not limitation, such computer-readable storage media may comprise RAM, ROM, EEPROM, flash memory or other memory devices, CD-ROM or other optical disc storage, magnetic disc storage or other magnetic storage devices, or any other medium that can be used to carry or store desired program code in the form of instructions or data structures and that can be accessed by a computer. Disc and disk, as used herein, include compact disc (CD), laser disc, optical disc, digital versatile disc (DVD), floppy disk, and bluray disc (BD). Further, a propagated signal is not included within the scope of computer-readable storage media. Computer-readable media also includes communication media including any medium that facilitates transfer of a computer program from one place to another. A connection, for instance, can be a communication medium. For example, if the software is transmitted from a website, server, or other remote source using a coaxial cable, fiber optic cable,twisted pair, DSL, or wireless technologies such as infrared, radio, and microwave are included in the definition of communication medium. Combinations of the above should also be included within the scope of computer-readable media.

Alternatively, or in addition, the functionality described herein can be performed, at least in part, by one or more hardware logic components. For example, and without limitation, hardware logic components that can be used may include Field-programmable Gate Arrays (FPGAs), Program-specific Integrated Circuits (ASICs), Program-specific Standard Products (ASSPs), System-on-a-chip systems (SOCs). Complex Progrmmable Logic Devices (CPLDs), etc.

Although illustrated as a single system, it is to be understood that the computing device may be a distributed system. Thus, for instance, several devices may be in communication by way of a network connection and may collectively perform tasks described as being performed by the computing device.

Although illustrated as a local device it will be appreciated that the computing device may be located remotely and accessed via a network or other communication link (for example using a communication interface).

The term 'computer' is used herein to refer to any device with processing capability such that it can execute instructions. Those skilled in the art will realise that such processing capabilities are incorporated into many different devices and therefore the term 'computer' includes PCs, servers, mobile telephones, personal digital assistants and many other devices.

Those skilled in the art will realise that storage devices utilised to store program instructions can be distributed across a network. For example, a remote computer may store an example of the process described as software. A local or terminal computer may access the remote computer and download a part or all of the software to run the program. Alternatively, the local computer may download pieces of the software as needed, or execute some software instructions at the local terminal and some at the remote computer (or computer network). Those skilled in the art will also realise that by utilising conventional techniques known to those skilled in the art that all, or a portion of the software instructions may be carried out by a dedicated circuit, such as a DSP, programmable logic array, or the like.

It will be understood that the benefits and advantages described above may relate to one embodiment or may relate to several embodiments. The embodiments are not limited to those that solve any or all of the stated problems or those that have any or all of the stated benefits and advantages.

Any reference to 'an' item refers to one or more of those items. The term 'comprising' is used herein to mean including the method steps or elements identified, but that such steps or elements do not comprise an exclusive list and a method or apparatus may contain additional steps or elements.

As used herein, the terms "component" and "system" are intended to encompass computer-readable data storage that is configured with computer-executable instructions that cause certain functionality to be performed when executed by a processor. The computer-executable instructions may include a routine, a function, or the like. It is also to be understood that a component or system may be localized on a single device or distributed across several devices.

Further, as used herein, the term "exemplary" is intended to mean "serving as an illustration or example of something".

Further, to the extent that the term "includes" is used in either the detailed description or the claims, such term is intended to be inclusive in a manner similar to the term "comprising" as "comprising" is interpreted when employed as a transitional word in a claim.

The figures illustrate exemplary methods. While the methods are shown and described as being a series of acts that are performed in a particular sequence, it is to be understood and appreciated that the methods are not limited by the order of the sequence. For example, some acts can occur in a different order than what is described herein. In addition, an act can occur concurrently with another act. Further, in some instances, not all acts may be required to implement a method described herein.

Moreover, the acts described herein may comprise computer-executable instructions that can be implemented by one or more processors and/or stored on a computer-readable medium or media. The computer-executable instructions can include routines, sub-routines, programs, threads of execution, and/or the like. Still further, results of acts of the methods can be stored in a computer-readable medium, displayed on a display device, and/or the like.

The order of the steps of the methods described herein is exemplary, but the steps may be carried out in any suitable order, or simultaneously where appropriate. Additionally, steps may be added or substituted in, or individual steps may be deleted from any of the methods without departing from the scope of the subject matter described herein. Aspects of any of the examples described above may be combined with aspects of any of the other examples described to form further examples without losing the effect sought.

It will be understood that the above description of a preferred embodiment is given by way of example only and that various modifications may be made by those skilled in the art. What has been described above includes examples of one or more embodiments. It is, of course, not possible to describe every conceivable modification and alteration of the above devices or methods for purposes of describing the aforementioned aspects, but one of ordinary skill in the art can recognize that many further modifications and permutations of various aspects are possible. Accordingly, the described aspects are intended to embrace all such alterations, modifications, and variations that fall within the scope of the appended claims.

## Claims

1. A computer-implemented method of training a machine learning model to learn ligand binding similarities between protein binding sites for use in drug discovery, the method comprising:
inputting to the machine learning model:
a representation of a first binding site;
a representation of a second binding site, wherein the representations of the first and second binding sites comprise structural information,
wherein the structural information relates to three-dimensional structure of the binding sites and the structural information comprises volumetric information,
wherein the representations of the first and second binding sites each comprise an encoded three-dimensional grid of voxels, each voxel being associated with an occupancy value indicating whether an atom is present; and
a label comprising an indication of ligand binding similarity between the first binding site and the second binding site;
outputting from the machine learning model a similarity indicator based on the representations of the first and second binding sites;
performing a comparison between the similarity indicator and the label; and
updating the machine learning model based on the comparison.

2. The computer-implemented method of claim 1, wherein each voxel is associated with a further value indicating a property selected from the set of hydrophobicity, aromaticity, acceptance or donation of a hydrogen bond, positive or negative ionizability, and being metallic.

3. The computer-implemented method of any preceding claim, wherein the machine learning model comprises a neural network and wherein the neural network comprises one or more convolutional layers.

4. The computer-implemented method of claim 3, wherein the neural network comprises one or more max-pooling layers; and/or wherein the neural network comprises a steerable three-dimensional convolutional neural network.

5. The computer-implemented method of any of claim 3 or 4, wherein the neural network comprises a deep learning neural network.

6. The computer-implemented method of any preceding claim, wherein performing the comparison comprises minimising a loss function.

7. The computer-implemented method of claim 6, wherein updating the machine learning model comprises performing back propagation using the minimised loss function.

8. The computer-implemented method of claim 6 or 7, wherein the loss function comprises a contrastive loss representing a loss between the similarity indicator and the label.

9. The computer-implemented method of claim 6, 7, or 8, wherein the loss function comprises a triplet loss based on a pair of binding sites, a reference binding site and the label.

10. The computer-implemented method of any preceding claim, comprising jittering the binding sites in input space.

11. The computer-implemented method of any preceding claim, wherein the label comprises a binary value indicating whether the first and second binding sites bind structurally similar ligands.

12. A neural network model obtained from a computer implemented method according to any one of claims 3 to 5.

13. An apparatus comprising a processor, a memory unit and a communication interface, wherein the processor is connected to the memory unit and the communication interface, wherein the processor and memory are configured to implement the computer-implemented method according to any one of claims 1 to 11.

14. A computer-readable medium comprising data or instruction code which, when executed by a computer, causes the computer to implement the computer-implemented method of any of claims 1 to 11.

## Patentansprüche

1. Computerimplementiertes Verfahren zum Trainieren eines maschinellen Lernmodells, die Ligandenbindungsähnlichkeiten zwischen Proteinbindungsstellen zur Verwendung bei der Wirkstoffentdeckung zu erlernen, wobei das Verfahren umfasst:
Eingabe in das maschinelle Lernmodell:
einer Darstellung einer ersten Bindungsstelle;
einer Darstellung einer zweiten Bindungsstelle, wobei die Darstellungen der ersten und zweiten Bindungsstelle strukturelle Informationen umfassen,
wobei die strukturellen Informationen eine dreidimensionale Struktur der Bindungsstellen betreffen und die strukturellen Informationen volumetrische Informationen umfassen,
wobei die Darstellungen der ersten und zweiten Bindungsstelle jeweils ein codiertes dreidimensionales Voxelraster umfassen, wobei jedes Voxel mit einem Besetzungswert verbunden ist, der anzeigt, ob ein Atom vorhanden ist; und
einer Kennzeichnung, umfassend eine Anzeige einer Ligandenbindungsähnlichkeit zwischen der ersten Bindungsstelle und der zweiten Bindungsstelle;
Ausgabe aus dem maschinellen Lernmodell eines Ähnlichkeitsindikators basierend auf den Darstellungen der ersten und zweiten Bindungsstelle;
Durchführung eines Vergleichs zwischen dem Ähnlichkeitsindikator und der Kennzeichnung; und
Aktualisierung des maschinellen Lernmodells basierend auf dem Vergleich.

2. Computerimplementiertes Verfahren nach Anspruch 1, wobei jedes Voxel mit einem weiteren Wert verbunden ist, der eine Eigenschaft anzeigt, die ausgewählt ist aus der Gruppe bestehend aus Hydrophobizität, Aromatizität, Annahme oder Spende einer Wasserstoffbrückenbindung, positiver oder negativer Ionisierbarkeit und der Eigenschaft, metallisch zu sein.

3. Computerimplementiertes Verfahren nach einem vorhergehenden Anspruch, wobei das maschinelle Lernmodell ein neuronales Netz umfasst und wobei das neuronale Netz eine oder mehrere Faltungsschichten umfasst.

4. Computerimplementiertes Verfahren nach Anspruch 3, wobei das neuronale Netz eine oder mehrere Max-Pooling-Schichten umfasst; und/oder wobei das neuronale Netz ein lenkbares, dreidimensionales, faltendes neuronales Netz umfasst.

5. Computerimplementiertes Verfahren nach einem von Anspruch 3 oder 4, wobei das neuronale Netz ein tief lernendes neuronales Netz umfasst.

6. Computerimplementiertes Verfahren nach einem vorhergehenden Anspruch, wobei die Durchführung des Vergleichs die Minimierung einer Verlustfunktion umfasst.

7. Computerimplementiertes Verfahren nach Anspruch 6, wobei eine Aktualisierung des maschinellen Lernmodells die Durchführung einer Rückpropagierung unter Verwendung der minimierten Verlustfunktion umfasst.

8. Computerimplementiertes Verfahren nach Anspruch 6 oder 7, wobei die Verlustfunktion einen kontrastiven Verlust umfasst, der einen Verlust zwischen dem Ähnlichkeitsindikator und der Kennzeichnung repräsentiert.

9. Computerimplementiertes Verfahren nach Anspruch 6, 7 oder 8, wobei die Verlustfunktion einen Triplettverlust basierend auf einem Paar von Bindungsstellen, einer Referenzbindungsstelle und der Kennzeichnung, umfasst.

10. Computerimplementiertes Verfahren nach einem vorhergehenden Anspruch, umfassend das Jittern der Bindungsstellen im Eingaberaum.

11. Computerimplementiertes Verfahren nach einem vorhergehenden Anspruch, wobei die Kennzeichnung einen binären Wert umfasst, der anzeigt, ob die erste und zweite Bindungsstelle strukturell ähnliche Liganden binden.

12. Neuronales Netzmodell, das von einem computerimplementierten Verfahren nach einem der Ansprüche 3 bis 5 erhalten wird.

13. Vorrichtung, umfassend einen Prozessor, eine Speichereinheit und eine Kommunikationsschnittstelle, wobei der Prozessor mit der Speichereinheit und der Kommunikationsschnittstelle verbunden ist, wobei der Prozessor und der Speicher so konfiguriert sind, dass sie das computerimplementierte Verfahren nach einem der Ansprüche 1 bis 11 implementieren.

14. Computerlesbares Medium, umfassend Daten- oder Befehlscode, der bei Ausführung durch einen Computer den Computer veranlasst, das computerimplementierte Verfahren nach einem der Ansprüche 1 bis 11 zu implementieren.

## Revendications

1. Procédé mis en oeuvre par l'informatique d'entraînement d'un modèle d'apprentissage machine pour apprendre des similarités de liaison de ligand entre des sites de liaison de protéine pour utilisation dans la découverte de médicament, le procédé comprenant :
entrer dans le modèle d'apprentissage machine :
une représentation d'un premier site de liaison ;
une représentation d'un deuxième site de liaison, dans lequel les représentations du premier et du deuxième site de liaison comprennent des informations structurelles,
dans lequel les informations structurelles se rapportent à une structure tridimensionnelle des sites de liaison et les informations structurelles comprennent des informations volumétriques,
dans lequel les représentations du premier et du deuxième site de liaison comprennent chacune une grille tridimensionnelle codée de voxels, chaque voxel étant associé à une valeur d'occupation indiquant si un atome est présent ; et
une étiquette comprenant une indication de similarité de liaison de ligand entre le premier site de liaison et le deuxième site de liaison ;
sortir du modèle d'apprentissage machine un indicateur de similarité basé sur les représentations du premier et du deuxième site de liaison ;
faire une comparaison entre l'indicateur de similarité et l'étiquette ; et
actualiser le modèle d'apprentissage machine sur la base de la comparaison.

2. Procédé mis en oeuvre par l'informatique selon la revendication 1, dans lequel chaque voxel est associé à une valeur supplémentaire indiquant une propriété sélectionnée parmi l'ensemble d'hydrophobie, d'aromaticité, d'acceptation ou de donation d'une liaison hydrogène, d'aptitude ionisable positive ou négative et d'être métallique.

3. Procédé mis en oeuvre par l'informatique selon l'une quelconque des revendications précédentes, dans lequel le modèle d'apprentissage machine comprend un réseau neuronal et dans lequel le réseau neuronal comprend une ou plusieurs couches convolutives.

4. Procédé mis en oeuvre par l'informatique selon la revendication 3, dans lequel le réseau neuronal comprend une ou plusieurs couches de max-pooling ; et/ou dans lequel le réseau neuronal comprend un réseau neuronal convolutif tridimensionnel orientable.

5. Procédé mis en oeuvre par l'informatique selon l'une quelconque des revendications 3 ou 4, dans lequel le réseau neuronal comprend un réseau neuronal d'apprentissage profond.

6. Procédé mis en oeuvre par l'informatique selon l'une quelconque des revendications précédentes, dans lequel faire la comparaison comprend minimiser une fonction de perte.

7. Procédé mis en oeuvre par l'informatique selon la revendication 6, dans lequel actualiser le modèle d'apprentissage machine comprend effectuer une rétropropagation en utilisant la fonction de perte minimisée.

8. Procédé mis en oeuvre par l'informatique selon la revendication 6 ou 7, dans lequel la fonction de perte comprend une perte contrastive représentant une perte entre l'indicateur de similarité et l'étiquette.

9. Procédé mis en oeuvre par l'informatique selon la revendication 6, 7 ou 8, dans lequel la fonction de perte comprend une perte en triplet basée sur une paire de sites de liaison, un site de liaison de référence et l'étiquette.

10. Procédé mis en oeuvre par l'informatique selon l'une quelconque des revendications précédentes, comprenant faire sautiller les sites de liaison dans l'espace d'entrée.

11. Procédé mis en oeuvre par l'informatique selon l'une quelconque des revendications précédentes, dans lequel l'étiquette comprend une valeur binaire indiquant si le premier et le deuxième site de liaison lient des ligands structurellement similaires.

12. Modèle de réseau neuronal obtenu d'un procédé mis en oeuvre par l'informatique selon l'une quelconque des revendications 3 à 5.

13. Appareil comprenant un processeur, une unité de mémoire et une interface de communication, dans lequel le processeur est connecté à l'unité de mémoire et à l'interface de communication, dans lequel le processeur et l'unité de mémoire sont configurés pour mettre en oeuvre le procédé mis en oeuvre par l'informatique selon l'une quelconque des revendications 1 à 11.

14. Support lisible par ordinateur comprenant des données ou du code d'instruction qui, lorsque exécutés par un ordinateur, font que l'ordinateur met en oeuvre le procédé mis en oeuvre par l'informatique selon l'une quelconque des revendications 1 à 11.
